# EUROPEAN PATENT SPECIFICATION

(11) **EP 0 783 339 B1**
(45) Date of publication and mention of the grant of the patent: **11.12.2002**
(21) Application number: 95933985.4
(22) Date of filing: 11.10.1995
(51) Int. Cl.: A61M 27/00, A61M 1/00

(54) **Improved wound drainage apparatus**
Verbesserte Vorrichtung zur Wunddrainage
Perfectionnement d'un appareil de drainage de plaie

(30) Priority: 11.10.1994 AU PM871294; 04.01.1995 AU PM038695
(43) Date of publication of application: 16.07.1997
(73) Proprietor: RESEARCH MEDICAL PTY. LTD., Leederville, W.A. 6007 (AU)
(72) Inventor: DIXON, Michael, Fremantle, W.A. 6160 (AU); STUBBER, Raymond, Lawrence, Sorrento, W.A. 6020 (AU)
(74) Representative: Newby, Martin John
(86) International application number: AU9500674
(87) International publication number: WO96011031

(56) References cited:
- EP-A- 0 113 541
- WO-A-88/05319
- AU-A- 1 967 583
- AU-A- 3 284 493
- FR-A- 2 560 770
- GB-A- 2 077 600
- US-A- 3 086 528
- US-A- 3 659 605
- US-A- 3 830 238
- US-A- 4 303 072
- US-A- 4 681 571
- US-A- 4 718 895
- US-A- 5 423 780

## Description

### Field of the Invention

This invention relates to a wound drainage apparatus where sub-atmospheric pressure (partial vacuum) is employed to assist the drainage of wound fluid from a body part. The function of wound drainage is to promote rapid and efficient healing of post-operative wounds.

### Background of the Invention

A wide range of equipment has been proposed to assist in the draining of fluid from wounds, particularly wounds resulting from surgery or accidents. One type of known construction employs a bellows type container that can be reduced in volume against the pressure of internal springs or by the force of deformation of the container material to provide a vacuum source.

These types of construction exhibit several major limitations one being that to achieve the desired level of vacuum, the container must have a high degree of resilience to develop a vacuum of the order of several psi (where 1 psi = 6·89 kPa). This is somewhat impractical in terms of cost effective manufacture and it is not possible to effectively control or maintain the desired vacuum level for efficient drainage. Further, as only a small degree of vacuum can be created, it dissipates rapidly as the container fills with fluid. Also, it is normally very difficult, if not impossible, to monitor accurately the rate at which the fluid is collected.

Another known type of equipment for the extraction and collection of wound fluid employs a disposable container connected to a re-useable electronically driven pump, and thus require elaborate and expensive electronics, a pump and rechargeable batteries or a power supply. The inherent complexities of this type of device entails significant additional cost for initial purchase and for maintenance of the pump and controller. While the fluid containers are disposable, special provisions must be made to ensure that the non-disposable components, such as the pump assembly, are protected from contamination by the drained wound fluid.

GB-A-2 077 600 discloses a chest drainage apparatus in which body fluid is received in a container via a conduit connected to the patient. The flow of body fluid to the container is controlled by a valve connected to a source of suction although the body fluid does not pass through the valve in use.

Yet a further known type of wound fluid collector is that employing a pre-evacuated container which use volatile liquids such as pentane and hexane to achieve the required level of vacuum. However these do not provide a capability to vary or otherwise control the level of vacuum, and if a leak occurs which dissipates the vacuum there is no ability to re-establish the vacuum. These systems often operate at a higher degree of vacuum than is ideally desired for optimum drainage in many situations.

There is a variant of this latter form of wound fluid collector which use an elaborate, separate, non-adjustable regulator of the flexible throttled tube type which can be attached to the drain bottle. This does help to avoid an overly strong suction, but still has the other shortcomings of the non-regulated system and additionally there is the substantial added cost of the regulator, which cannot be re-used for another patient, as the regulator becomes contaminated by the drained wound fluids during use.

### Objects and Summary of the Invention

The object of this invention is to provide an improved apparatus for effecting closed wound drainage especially in regard to a cost effectiveness, disposability, and reliability and simplicity in operation.

According to the present invention there is provided a wound discharge apparatus as claimed in the ensuing claim 1.

Preferably in use the level of vacuum is maintained in the container is greater than that existing in the conduit and the opening of the valve means establishes a fluid flow from the conduit through the valve means into the container to remove fluid from the conduit and so maintain the desired level of vacuum in the conduit.

Conveniently adjustment of the valve means adjusts a selected level of vacuum to be maintained in the conduit is selectable within a desired preset range.

Conveniently, the valve means is adjustable to respond to a level of vacuum between zero to a chosen maximum degree of vacuum thereby enabling the level of vacuum in the conduit to be adjusted to suit the prevailing wound condition.

Preferably, the valve means is operated by a diaphragm means exposed to atmospheric pressure on one side and to the level of vacuum existing in the conduit on the other side. An adjustable force means may be arranged to apply a force to the diaphragm in opposition to the atmospheric pressure. The difference between the force applied by the adjustable force means and the force generated by the atmospheric pressure is the force required to be applied by the level of vacuum in the conduit to close the valve means to interrupt communication between the container and the conduit, and consequently the level of vacuum existing to promote withdrawal of fluid from the wound.

Thus variations in the extent of the force applied by the adjustable force means controls the level of vacuum in the conduit that effects withdrawal of the fluid from the wound. The higher the force applied by the adjustable force means, in opposition to the force of atmospheric pressure on the diaphragm, the lower is the sub-atmospheric pressure maintained in the conduit to withdraw wound fluid, and vice versa.

The invention will be more readily understood from the following description of one practical arrangement of the wound drainage system and apparatus as illustrated in the accompanying drawings.

In the drawings:
Figure 1 is an isometric view of the overall configuration of the wound draining device;
Figure 2 is a plan view of the top of the draining device;
Figure 3 is a section on line A-A of Figure 2;
Figure 4 is a partial section on line B-B of Figure 2.
Figure 5 is an isometric view of the overall configuration of an alternative arrangement of the draining device.
Figure 6 is a sectional view of the regulator valve shown in Figure 5.

### Detailed Description of the Embodiment

The drainage apparatus includes a drainage container 10 with a cap 15 in which the functional components of the system are incorporated. The functional components include a drain tube 22, the free end (not shown) of which in use communicates with the wound site in the known conventional manner, is connected at the other end by a bayonet type fitting 20 to the cap 15 of the container 10 and incorporates a check valve 35 that will not permit flow outward from the container 10 into the tube 22.

The tube 50 within the container 10 extends from the check valve 35 to the underside of the regulator valve 40. The spigot 25, incorporating a further check valve 26, is provided for connection to a conventional in situ hospital vacuum system, thereby enabling evacuation of the drainage container 10. The resilient cone 30 functions as a diaphragm to be deflected into the container 10 to visually indicate the presence of at least partial vacuum in the container 10.

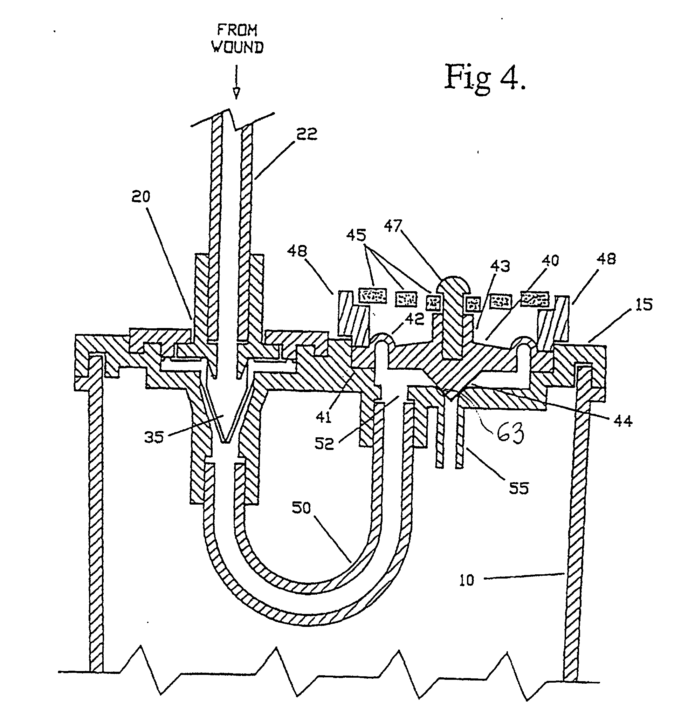

Referring to Figure 4, the regulator valve 40 consists of a moulded elastomer disc sealably secured at the perimeter 41 thereof to the cap, and has a concentric annular compliant section 42 a threaded boss 43 at the centre of the upper face, and a central valve section 44 on the lower face. In the free position of the regulator valve 40 the valve section 44 will engage the seat 63 in the upper end of the drain tube 50 to close the tube. Above the regulator valve 40 is a rotatable compliant or resilient element 45 which when rotated will adjust the force applied to the regulator valve 40, in opposition to atmospheric pressure applied externally thereto.

The magnitude of force applied by the compliant element 45 is selectively adjustable by a rotational movement of the compliant element 45 in the peripheral support 48 thereof. The degree of rotation is indicated by markings 70, resulting in an indication of the up force applied to the regulator valve 40. The higher the force applied by the compliant element 45, the higher the level of vacuum maintained in the tube 50. This level can be further varied through the use of different grades of compliant component for different applications of the drainage system.

When wound fluid is aspirated through the tube 50 to the underside of the regulator valve 40, it is then vented through the spigot 55 into the interior of the drain container 10. The drain container has an inverted conical base 60, which allows for an expanded vertical scale of the level indicator 65 for more accurate measurement of the initial flow of wound fluid. The inverted conical shape is also advantageous in that it improves the resistance of the container to deformation due to the presence of vacuum therein.

It is well established from clinical practice that healing of post-operative wounds is assisted by proper drainage of excess fluid from within the wound site. The ideal degree of suction to be applied will vary from case to case and from time to time during the healing process. However, it can be said that the degree of suction is generally agreed to desirable lie in the range between zero and 15 kPa below atmospheric pressure.

Wall mounted reticulated vacuum systems commonly provided in hospitals are typically set between 65 and 80 kPa below atmospheric pressure (101.3 kPa), which is much too strong a suction to be used for continuous wound drainage.

The level of vacuum to be applied to the wound site by the presently proposed system is controllable through the regulator valve 40, which controls the pressure level in the wound drain tube 22. The pressure in the drainage container 10 and at the point where the spigot tube 55 meets the valve seat 63 will typically be around 70 kPa below atmospheric, immediately after evacuation by the hospital reticulated vacuum system. As the regulator valve 40 in this state is closed, no fluid can flow until there is an upward force applied to the regulator valve 40.

If gravity drainage without suction assistance is required, the upward force applied to the regulator valve can be set to zero. Independent of the degree of vacuum in the container 10, any pressure rise in the drain line 22, due to newly produced wound fluid, will be sufficient to open the valve seating of the valve section 44 on the valve seat 63 and allow fluid to flow into the container through the drain spigot tube 55.

Where suction assistance is required, this is achieved by the compliant element 45, secured to the boss 43 of the regulator valve 40 by a screw 47, which is induced to flex by rotation against a circular ramp of the peripheral support 48. The degree of flex induced is related to the degree of rotation, and can be read against the calibrated scale 70. The upward force resulting from the flexion of the compliant member 45 opens the regulator valve 40, breaking the seal of the valve section 44 with the valve seat 63 and allowing fluid to be drawn through the spigot tube 55 into the drain container 10, thereby lowering the pressure in the area below the regulator valve, and through the orifice 52, equally lowering the pressure in the connecting tube 50 and the drain tube 22.

As fluid is passed into the drainage container 10, the pressure will reduce until a level is reached where the downward force on the lower surface of the regulator valve 40 opposing the upward flexion force applied by the compliant element 45, reaches a point of equilibrium. The regulator valve 40 will then return to its closed position. The regulator valve needs only to move small fractions of a millimetre in the transition between closed and open.

The state of equilibrium where the valve is closed can only be disturbed by either changing the upward force applied to the regulator valve 40 by the compliant element 45, thereby requiring an increased level of vacuum in the system to restore equilibrium, or by the production of a flow of wound fluid into the drain tube 22. The added volume of fluid will dissipate the level of vacuum in the drain line 22 and connecting tube 50, hence lowering the downward force on the regulator valve 40. This in turn will allow flow of wound fluid into the drain container 10, thereby increasing the level of vacuum until equilibrium is again restored.

As fluid flows into the drain container 10, the level of vacuum therein will gradually dissipate. A container of 0·6m³ (600 ml), evacuated to minus 75 kPa, will, after draining 0·4m³ (400 ml) of wound fluid, fall to minus 10 kPa, and upon draining 0·45 m³ (450 ml) the vacuum will be completely dissipated.

The regulated level of vacuum in the drain line 22 is controlled by the effective size of the regulator disc 42 measured at the centre line of the annular compliant element 45 and the consequent force applied from a given pressure differential, balanced against the degree of force applied by the compliant element 45. The regulated pressure will not be adversely influenced by variation in the level of vacuum in the drain container 10 if the cross sectional area of the spigot tube 55 is kept to less than 2% of the effective area of the regulator disc 42.

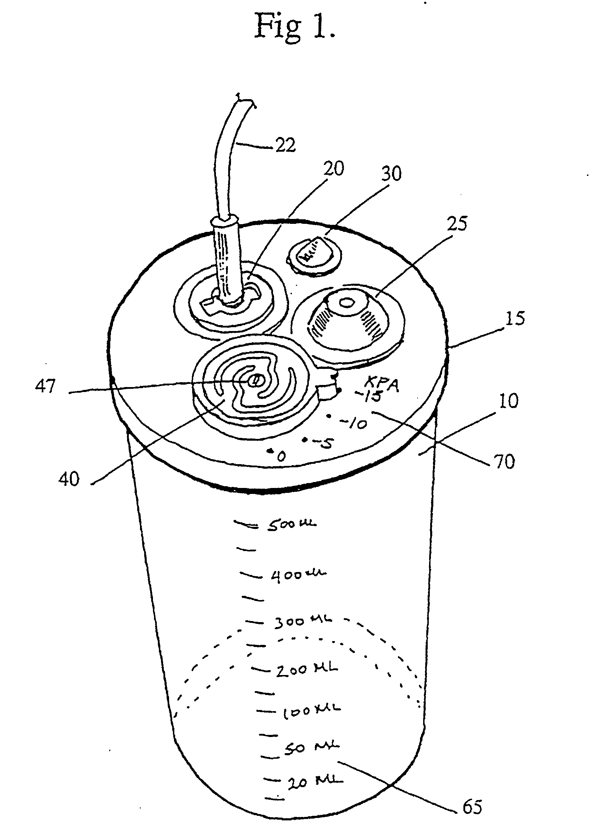

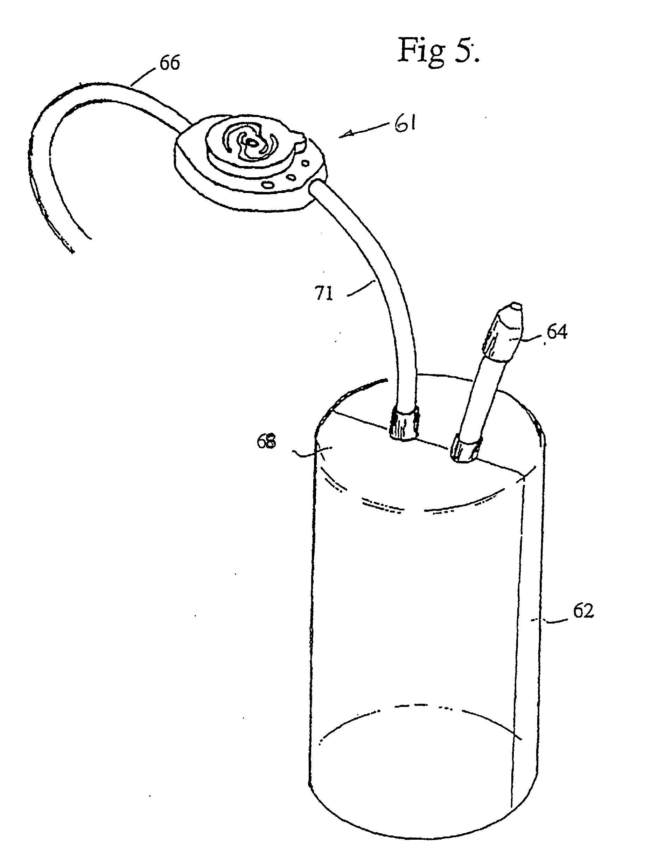

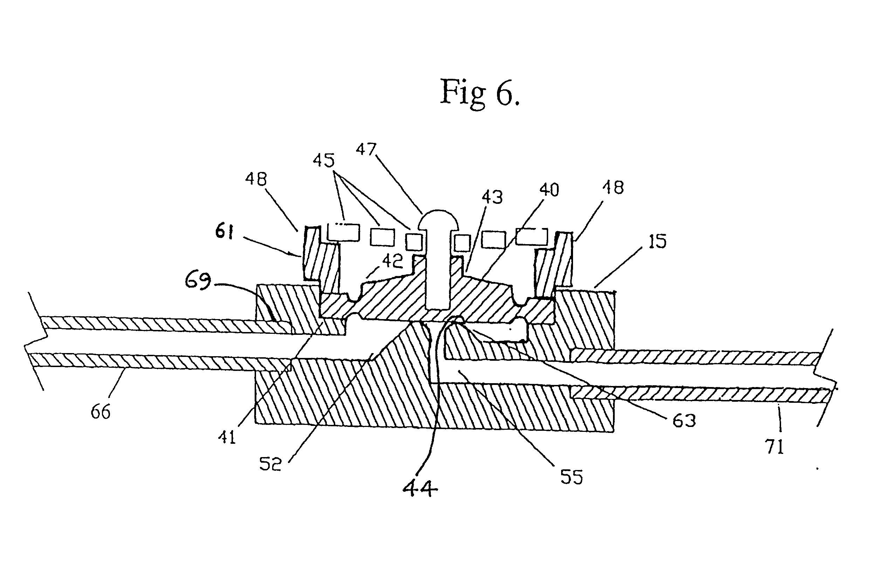

There is shown in Figures 5 and 6 an alternative form of the wound drainage apparatus in which a regulator valve 61 is constructed independently of the container in which the wound fluid is collected. By separating the regulator valve from the container, the container can be of a simplified construction that can be effectively sterilised and therefore be re-used numerous times. This is a substantial commercial advantage compared with the integrally constructed container and regulator valve unit described with reference to Figures 1 to 4, which cannot be effectively sterilised, and hence must be totally discarded after a single use.

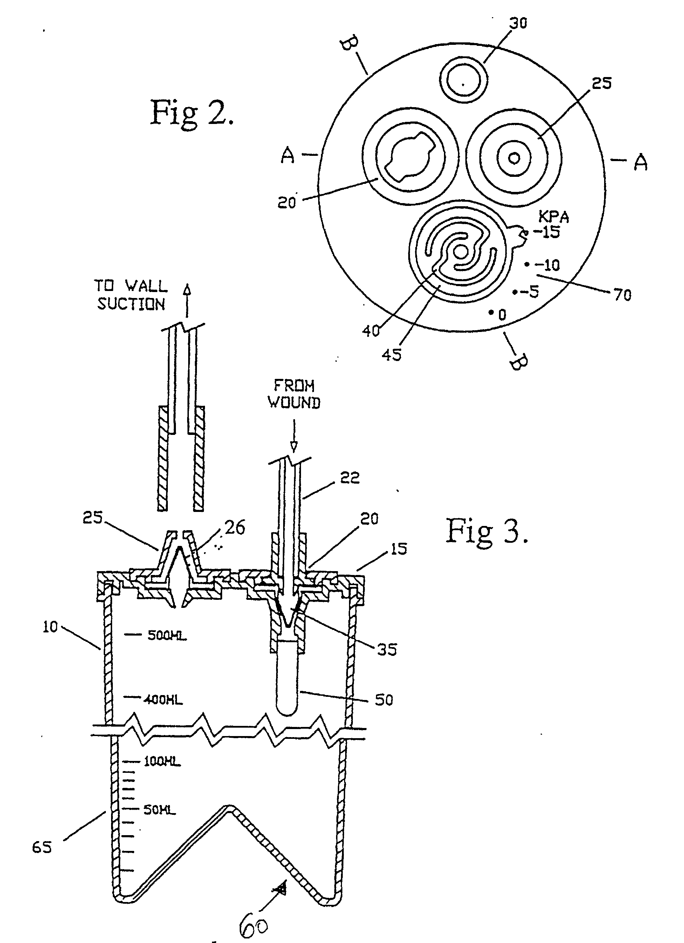

As seen in Figure 5, the container 62 is of a simple cylindrical form with a removable closure or cap 68, and may have an inverted conical base similar to the conical base 60 of the container shown in Figure 3. The container cap 68 is removable for discarding of the fluid collected therein and for sterilisation between successive uses. The coupling 64 provided in the cap 68 is connectable to the hospital reticulated vacuum system to enable initial establishment of the required level of vacuum in the container.

The independent regulator 61 is connected to the container 62 by the tube 71 and to the wound by a conventional wound drainage tube portion shown at 66.

The construction and operation of the regulator 61 is substantially the same as the regulator 40 previously described. The regulator disc 42, compliance element 45 and screw 47, and the operation and adjustment thereof, are each identical to that previously described with reference to Figures 3 and 4, and have therefore been identified by the same reference numeral. The description thereof will not be repeated here. The differences from the regulator 40 reside in the wound fluid drain tube 66 being received in sealed relation in the cavity 69 to communicate with the cavity 52 immediately upstream of the valve 44. Also the passage 55, downstream of the valve 44, communicates directly with the transfer tube 71 via which wound fluid is transferred to an independent container (not shown) but would basically be the same as the container 10, but without the regulator valve incorporated therein.

This construction employing separate regulator valve and fluid collection container, providing the ability for multiple use of the container significantly improves the economics of the complete wound drainage unit.

In the regulator valve described with reference to Figures 2, 3 and 4 or Figures 5 and 6, the compliant member 45 is in the form of a disk which is rotated against a circular ramp to vary the degree of flexion induced, thus exerting an upward force on the regulator valve 40. The same functional effect could also be achieved by employing other means such as:
a compliant beam secured to the cap 48 at one end, and to the regulator valve 40 at the other, with a central screw or ramp device to lift the beam's centre point, thereby applying a controlled upward force to the regulator valve;
a compliant beam secured to the regulator valve at one end, with a fulcrum in the centre and a screw or ramp device to press down on the other end, which would also apply a controlled upward force to the regulator valve;
a tension spring, one end attached to the upper side of the regulator valve, the other to an adjustably moveable anchor point, allowing variable tension force to be generated in the spring, thus applying a controlled upward force to the regulator valve.

The materials envisioned for the construction of the invention would favour appropriate grades of mouldable plastics, but need not be limited to these. Other materials such as glass, metals and rubber could equally be employed as and where cost and performance dictated.

## Claims

1. A wound drainage apparatus for withdrawing wound fluid from a body part, comprising a conduit (22,50) connectable at one end to said body part, a container (10) for collecting wound fluid delivered from the body part by said conduit (22,50), and a valve means (40,61) providing selective communication between said conduit (22,50) and said container (10) for controlling the delivery of wound fluid to the container, **characterised in that** said valve means (40,61) is located such that wound fluid drained by said conduit (22,50) passes through said valve (40,61) to said container (10), and which, in use, is adapted to open against externally applied atmospheric pressure to allow wound fluid to drain via said valve (40,61) to said container when the level of vacuum, in said conduit (22,50) falls below a selected value due to the presence of wound fluid in said conduit (22,50), and to close due to the force of the externally applied atmospheric pressure when the level of vacuum in said conduit is re-established at or above the selected value following drainage of wound fluid via said valve means (40,61) to said container (10).

2. A wound drainage apparatus according to claim 1, **characterised in that** the valve means (40,61) includes an elastomeric disc having an annular compliant section (42), a valve section (44) for engaging a seat (63), a rotatable annular compliant or resilient regulator element (45) which, in use, is rotated against a circular ramp to vary the degree of flexion induced to the valve means (40,61) thereby providing force adjustment for the valve means (40,61) in opposition to the externally applied atmospheric pressure.

3. A wound drainage apparatus according to claim 1 or 2, **characterised in that** the container (10) is adapted to be detachably connectable to an in situ vacuum source for evacuating said container (10).

4. A wound drainage apparatus according to claim 3, **characterised in that** the container (10) includes a check valve (26) for maintaining a state of evacuation in said container (10) when detachably disconnected from said in situ vacuum source.

5. A wound drainage apparatus according to any one of the preceding claims, **characterised in that** said valve means (40,61) is detachably connectable to said container (10).

6. A wound drainage apparatus according to any one of the preceding claims, **characterised in that** said valve means (61) is detachably connectable to said container (10) by a delivery conduit (71).

7. A wound drainage apparatus according to claim 4, **characterised in that** the conduit (66), valve means (61) and delivery conduit (71) are an integral assembly detachably connectable to said container (10).

8. A wound drainage apparatus according to any one of the preceding claims, **characterised in that** a check valve (35) is provided downstream of said valve means (40,61) to prevent outward flow from said container (10) to said conduit (22,50).

9. A wound drainage apparatus according to any one of the preceding claims, **characterised in that** adjustment of said valve means (40,61) adjusts a selected level of vacuum in the conduit within a specific range.

## Patentansprüche

1. Wunddrainagevorrichtung zum Abziehen von Wundsekret von einem Körperteil, die eine an einem Ende mit dem Körperteil verbindbare Leitung (22, 50), einen Behälter (10) zum Sammeln des vom Körperteil durch die Leitung (22, 50) angelieferten Wundsekrets und ein Ventilmittel (40, 61) umfasst, das zur Steuerung der Anlieferung von Wundsekret zum Behälter (10) für eine gezielte Verbindung zwischen der Leitung (22, 50) und dem Behälter sorgt, **dadurch gekennzeichnet, dass** das Ventilmittel (40, 61) so angeordnet ist, dass von der Leitung (22, 50) abgeführtes Wundsekret durch das Ventil (40, 61) zu dem Behälter (10) fließt, und das Ventilmittel im Gebrauch gegen extern ausgeübten atmosphärischen Druck öffnen kann, damit Wundsekret über das Ventil (40, 61) in den Behälter ablaufen kann, wenn die Höhe des Vakuums in der Leitung (22, 50) aufgrund der Anwesenheit von Wundsekret in der Leitung (22, 50) unter einen gewählten Wert abfällt, und aufgrund der Kraft des extern ausgeübten atmosphärischen Drucks schließen kann, wenn die Höhe des Vakuums in der Leitung
nach der Drainage von Wundsekret über das Ventilmittel (40, 61) in den Behälter (10) wieder auf dem oder über dem gewählten Wert liegt.

2. Wunddrainagevorrichtung nach Anspruch 1, **dadurch gekennzeichnet, dass** das Ventilmittel (40, 61) eine elastomere Scheibe mit einem ringförmigen nachgiebigen Abschnitt (42), einen Ventilabschnitt (44) zur Ineingriffnahme eines Sitzes (63) und ein drehbares, ringförmiges nachgiebiges oder elastisches Regelelement (45) umfasst, das im Gebrauch gegen eine kreisförmige Rampe gedreht wird, um den in das Ventilmittel (40, 61) eingeleiteten Biegungsgrad zu variieren und dadurch Krafteinstellung für das Ventilmittel (40, 61) gegen den extern ausgeübten atmosphärischen Druck bereitzustellen.

3. Wunddrainagevorrichtung nach Anspruch 1 oder 2, **dadurch gekennzeichnet, dass** der Behälter (10) zu seiner Evakuierung lösbar an eine lokale Vakuumquelle angeschlossen werden kann.

4. Wunddrainagevorrichtung nach
Anspruche 3, **dadurch gekennzeichnet, dass** der Behälter (10) ein Rückschlagventil (26) umfasst, um in dem Behälter (10) einen Evakuierungszustand aufrechtzuerhalten, wenn er lösbar von der lokalen Vakuumquelle getrennt ist.

5. Wunddrainagevorrichtung nach einem der vorhergehenden Ansprüche, **dadurch gekennzeichnet, dass** das Ventilmittel (40, 61) lösbar an den Behälter (10) angeschlossen werden kann.

6. Wunddrainagevorrichtung nach einem der vorhergehenden Ansprüche, **dadurch gekennzeichnet, dass** das Ventilmittel (61) über eine Lieferleitung (71) lösbar an den Behälter (10) angeschlossen werden kann.

7. Wunddrainagevorrichtung nach Anspruch 4, **dadurch gekennzeichnet, dass** die Leitung (66), das Ventilmittel (61) und die Lieferleitung (71) eine integrale Baugruppe sind, die lösbar an den Behälter (10) angeschlossen werden kann.

8. Wunddrainagevorrichtung nach einem der vorhergehenden Ansprüche, **dadurch gekennzeichnet, dass** dem Ventilmittel (40, 61) ein Rückschlagventil (35) nachgeschaltet ist, um Ausfluss aus dem Behälter (10) in die Leitung (22, 50) zu verhindern.

9. Wunddrainagevorrichtung nach einem der vorhergehenden Ansprüche, **dadurch gekennzeichnet, dass** durch Einstellung des Ventilmittels (40, 61) eine gezielte Höhe des Vakuums in der Leitung innerhalb eines bestimmten Bereichs eingestellt wird.

## Revendications

1. Appareil de drainage de plaie pour extraire du fluide d'une plaie d'une partie corporelle, comprenant un conduit (22, 50) pouvant être connecté à une extrémité à ladite partie corporelle, un récipient (10) pour recueillir le fluide de la plaie apporté depuis la partie corporelle par ledit conduit (22, 50), et un moyen de soupape (40, 61) assurant une communication sélective entre ledit conduit (22, 50) et ledit récipient (10) pour contrôler l'apport de fluide de la plaie au récipient, **caractérisé en ce que** ledit moyen de soupape (40, 61) est situé de telle sorte que le fluide de la plaie drainé par ledit conduit (22, 50) passe à travers ladite soupape (40, 61) jusqu'audit récipient (10), et, en fonctionnement, est adapté pour s'ouvrir à l'encontre de la pression atmosphérique appliquée extérieurement pour permettre au fluide de la plaie d'être drainé par le biais de ladite soupape (40, 61) jusqu'audit récipient lorsque le niveau de vide dans ledit conduit (22, 50) tombe en dessous d'une valeur sélectionnée en raison de la présence de fluide de la plaie dans ledit conduit (22, 50), et pour se fermer sous l'effet de la force de la pression atmosphérique appliquée extérieurement lorsque le niveau de vide dans ledit conduit est rétabli à ou au-dessus de la valeur sélectionnée suite au drainage du fluide de la plaie par le biais dudit moyen de soupape (40, 61) jusqu'audit récipient (10).

2. Appareil de drainage de plaie selon la revendication 1, **caractérisé en ce que** le moyen de soupape (40, 61) comporte un disque élastomère ayant une section souple annulaire (42), une section de soupape (44) pour engager un siège (63), un élément de régulation (45) rotatif annulaire souple ou résilient qui, en fonctionnement, est tourné à l'encontre d'une rampe circulaire pour faire varier le degré de flexion appliquée au moyen de soupape (40, 61) en fournissant ainsi un ajustement de la force pour le moyen de soupape (40, 61) s'opposant à la pression atmosphérique appliquée extérieurement.

3. Appareil de drainage de plaie selon la revendication 1 ou 2, **caractérisé en ce que** le récipient (10) est prévu pour pouvoir être connecté de manière détachable à une source de vide in situ pour évacuer ledit récipient (10).

4. Appareil de drainage de plaie selon la revendication, 3,
**caractérisé en ce que** le récipient (10) comporte un clapet de non retour (26) pour maintenir un état d'évacuation dans ledit récipient (10) lorsqu'il est déconnecté de manière détachable de ladite source de vide in situ.

5. Appareil de drainage de plaie selon l'une quelconque des revendications précédentes, **caractérisé en ce que** ledit moyen de soupape (40, 61) peut être connecté de manière détachable audit récipient (10).

6. Appareil de drainage de plaie selon l'une quelconque des revendications précédentes, **caractérisé en ce que** ledit moyen de soupape (61) peut être connecté de manière détachable audit récipient (10) par un conduit d'amenée (71).

7. Appareil de drainage de plaie selon la revendication 4, **caractérisé en ce que** le conduit (66), le moyen de soupape (61) et le conduit d'amenée (71) sont un ensemble intégral pouvant être connecté de manière détachable audit récipient (10).

8. Appareil de drainage de plaie selon l'une quelconque des revendications précédentes, **caractérisé en ce qu'**un clapet de non retour (35) est prévu en aval dudit moyen de soupape (40, 61) pour empêcher un écoulement vers l'extérieur depuis ledit récipient (10) vers ledit conduit (22, 50) .

9. Appareil de drainage de plaie selon l'une quelconque des revendications précédentes, **caractérisé en ce que** l'ajustement dudit moyen de soupape (40, 61) ajuste un niveau sélectionné de vide dans le conduit dans une plage spécifique.
